# EUROPÄISCHE PATENTSCHRIFT

(11) **EP 2 371 350 B1**
(45) Veröffentlichungstag und Bekanntmachung des Hinweises auf die Patenterteilung: **24.04.2013**
(21) Anmeldenummer: 10155548.0
(22) Anmeldetag: 04.03.2010
(51) Int. Cl.: A61K 8/44, A61K 8/04, A61K 9/00, A61K 9/107, A61K 31/17, A61K 9/12, A61P 17/02, A61P 17/12, A61K 8/06, A61L 26/00, A61K 8/19, A61K 33/38, A61K 45/06, A61Q 19/00, A61K 33/00, A61K 8/42

(54) **Schaumformulierungen zur Behandlung von Hauterkrankungen beim Tier**
Foam formulas for treating animal skin illnesses
Formulations à base de mousse pour le traitement de maladies de peau chez les animaux

(43) Veröffentlichungstag der Anmeldung: 05.10.2011
(73) Patentinhaber: Neubourg Skin Care GmbH & Co. KG, 48268 Greven (DE)
(72) Erfinder: Neubourg, Thomas, 59368 Werne (DE)
(74) Vertreter: Maiwald, Walter

(56) Entgegenhaltungen:
- EP-A1- 1 287 814
- EP-A1- 2 095 807
- WO-A1-2005/016329
- WO-A1-2009/036714
- WO-A2-99/08649
- WO-A2-2008/075207
- WO-A2-2008/155389
- WO-A2-2010/000348
- US-A1- 2004 156 874
- US-A1- 2009 041 680
- ATIYEH ET AL: "Effect of silver on burn wound infection control and healing: Review of the literature", BURNS, BUTTERWORTH HEINEMANN, GB, Bd. 33, Nr. 2, 3. Februar 2007 (2007-02-03), Seiten 139-148, XP005873104, ISSN: 0305-4179, DOI: DOI:10.1016/J.BURNS.2006.06.010

## Beschreibung

### Technisches Gebiet der Erfindung

Die vorliegende Erfindung betrifft topische Darreichungsformen, insbesondere Schaumformulierungen, zur Vorbeugung und/oder Behandlung einer Hauterkrankung bei nicht-menschlichen Säugetieren.

### Hintergrund der Erfindung

### 1. Die Haut von Säugetieren

Die Haut von Säugetieren besitzt eine Vielzahl von Funktionen. Sie umhüllt den Körper der Tiere und bildet die physikalische Barriere zur Außenwelt. Dabei bietet sie einen gewissen Schutz vor physikalischen und chemischen Noxen und verhindert das Eindringen von Mikroorganismen in den Körper. Für Wasser ist sie undurchdringlich und schützt dadurch vor Austrocknung. Eine wichtige Rolle spielt die Haut u.a. auch bei der Thermoregulation, und fungiert über die Schweißdrüsen als Exkretionsorgan.

Die tierische Haut besteht aus zwei Schichten, einer äußeren Epidermis (Oberhaut) und einer darunter liegenden Dermis (Lederhaut). Epidermis und Dermis bilden zusammen die Cutis, die auf der Subcutis (Unterhaut) ruht. Die Epidermis wird von einem mehrschichtigen Plattenepithel gebildet und ist Trägerin der Barrierefunktion der Haut. Je nach Beanspruchung ist sie mehr oder weniger dick. An der behaarten Haut ist die Epidermis nur mäßig, an der unbehaarten Haut jedoch sehr stark verhornt. Abkömmlinge der Epidermis sind Schweiß- und Talgdrüsen, sowie Haare.

Die Haut zahlreicher Säugetiere unterscheidet sich von menschlicher Haut u.a. durch die deutlich stärkere Körperbehaarung. Die Haardichte ist bei verschiedenen Tierarten und je nach Körperregion sehr verschieden. Bei dicht behaarten Tieren findet man einige tausend Haare pro cm² (z.B. Katze 25.000, Hund 5.000, Schaf 6000-8000 Haare pro cm²). Bei Rindern, Ziegen, und Pferden werden um 1000 Haare pro cm² gezählt (Anatomie für die Tiermedizin, Hrsg. F.-V. Salomon, H. Geyer, U. Gille; Enke Verlag, Stuttgart, 2005). Das Haarkleid von Säugetieren bezeichnet man auch als Fell oder Pelz.

### 2. Hauterkrankungen bei nicht-menschlichen Säugetieren

Ebenso wie der Mensch können auch nicht-menschliche Säugetiere an zahlreichen Hauterkrankungen leiden. Dazu zählen u.a. Wunden, die beispielsweise durch Verletzungen, Verbrennungen oder Verätzungen verursacht werden können, bakterielle Hautinfektionen oder nicht-infektiöse Hautentzündungen. Je nach Tierart und insbesondere Lebensumständen bzw. Haltung des Tieres treten bestimmte Hauterkrankungen und deren konkrete Erscheinungsform besonders häufig auf.

### Pyodermie

Als Pyodermie bezeichnet man eine brennende, eitrige Entzündung der Haut, die durch bakterielle Infektion verursacht wird. Verschiedene Hautschichten können betroffen sein. Nach Umfang der betroffenen Hautschichten unterscheidet man zwischen Oberflächenpyodermie, oberflächlicher Pyodermie und tiefer Pyodermie. Bei der Oberflächenpyodermie sind nur die obersten Schichten der Epidermis betroffen. Sonderformen sind Intertrigo (Hautfaltendermatitis), die Pyotraumatische Dermatitis, die bakterielle Übersiedelung der Haut und die mukokutane Pyodermie. Bei der oberflächlichen Pyodermie hingegen sind auch tiefere Schichten der Epidermis, vor allem die im Bereich der Haarfollikel betroffen; die Infektion bleibt aber oberhalb der Basallamina. Sonderformen sind die Impetigo, die bullöse Impetigo und die oberflächliche bakterielle Follikulitis. Bei der tiefen Pyodermie kommt es auch zu einer Infektion der Dermis oder sogar der Subkutis, z.B. als tiefe Follikulitis, Furunkulose, oder Zellulitis.

Pyodermien sind eine häufige Hauterkrankung bei Tieren, z.B. beim Hund, die in der Mehrzahl der Fälle durch Staphylokokken hervorgerufen werden und typischerweise als Sekundärerkrankung auftreten. Die klassischen Veränderungen sind durch Papeln, Pusteln und Krusten charakterisiert. Seltener werden u.a. Komedone, Schuppenbildung, und Haarausfall beobachtet.

### Mauke

Mauke ist eine weit verbreitete Hauterkrankung des Pferdes, die medizinisch als Fesselekzem bezeichnet wird. Die klassische Mauke tritt in erster Linie in der Fesselbeuge auf, kann sich aber als so genannte Raspe bis über das Karpal- oder Tarsalgelenk ausdehnen. Besonders betroffen sind Pferdebeine, deren Haut unpigmentiert ist (weiße Abzeichen). Außerdem sind Pferde mit starkem Fesselbehang häufiger betroffen, wie z.B. Friesen, Tinker und Kaltblüter. Diese Rassen besitzen genetisch bedingt häufig eine unnatürlich verdickte Haut im Bereich der Fessel. Aufgrund der zu starken Hornschichtbildung und dem gestörten Feuchtigkeitshaushalt durch den langen und dichten Behang entstehen Risse und Schäden in der Haut, die ein Einnisten von Erregern begünstigen. Prinzipiell kann aber jede Pferderasse betroffen sein.

Bei der Mauke handelt es sich um eine bakterielle Infektion der Haut. Über eine Eintrittspforte, meist kleine Schäden in der Haut, gelangen Keime in den Organismus. Wenn die Bedingungen (Feuchtigkeit, Wärme, gute Haftungsbedingungen) für den Erreger optimal sind, kann er sich vermehren, und dabei die Haut angreifen. Dabei bereitet er den Nährboden für weitere bakterielle Keime oder Hautparasiten wie zum Beispiel Milben. Meist lassen sich bei einer Tupferprobe gleich mehrerer Bakterienarten differenzieren. Im weiteren Verlauf der Mauke-Erkrankung können sich auch Hautpilze auf der vorgeschädigten Haut verankern und vermehren. Man spricht dann von einer Mischinfektion.

Das Immunsystem des Körpers reagiert mit einer Entzündungsreaktion. Je nach Stärke der Reaktion tritt wässriges (seröses) Entzündungssekret aus, das mit Bakterien, Blut, Schmutzpartikeln und Haaren eine Kruste bildet. Unter dieser Kruste können sich Bakterien, aber auch Pilze und Hautparasiten ungestört vermehren.

In frühen Stadien der Mauke-Erkrankung sind nur die obersten Schichten der Haut betroffen. Die Haut ist gerötet, schmerzhaft, und mit zum Teil verkrustetem eitrigem Sekret bedeckt. Mit fortschreitender Erkrankung werden die tieferen Schichten der Haut befallen. Die Krusten werden größer und flächiger. Sie enthalten neben den Entzündungssekreten abgestorbenen Epithelzellen, Haare, Schmutz und Bakterien. Die Wundflächen reißen bei Belastung immer wieder blutig auf. Es entstehen Falten und tiefe Risse in der Haut. Eine spontane Heilungstendenz dieser Risse und Falten ist nur selten zu erkennen ― die Mauke wird chronisch. Die Haut ist nun nicht mehr in der Lage, die Erreger selbst ausreichend zu bekämpfen. Oft wird die Haut dann flächig wund, erscheint rosarot mit feuchten entzündlichen Ausschwitzungen und nur noch vereinzelten Haarstummeln.

### Dermatitis digitalis (Mortellaro)

Die Dermatitis digitalis oder Mortellaro ist eine der bedeutsamsten Klauenerkrankungen bei Rindern. Ihre Ätiologie ist bis heute nicht hinreichend geklärt. Vermutlich spielen Bakterien eine wichtige Rolle in der Pathogenese. Zum Ausbruch der Erkrankung kommt es vermutlich nur bei Zusammenwirken verschiedener Risikofaktoren wie z.B. Feuchtigkeit oder mangelnde Stallhygiene.

Zu Beginn der Erkrankung werden epidermale Läsionen von nur wenigen Millimetern Durchmesser gesehen, die sich im weiteren Verlauf zu ulzerativen Hautveränderungen weiterentwickeln können. Der betroffene Hautbezirk wird leicht übersehen und ist oftmals erst nach Reinigung deutlich erkennbar, da er durch graugelblichen Detritus überdeckt ist. Dem Belag haftet ein eigentümlich fauligsüßlicher Geruch an. Am häufigsten wird die Dermatitis digitalis als eine umschriebene, kreisrunde oder längsovale ulzerative Hautentzündung beschrieben, die überwiegend im Bereich der Fesselbeuge, am Übergang der Haut zum Ballenhorn auftritt. Granulationsgewebe verleiht der Läsion einen roten, erdbeerartigen Aspekt. Die Dermatitis digitalis wird am häufigsten an den Hintergliedmaßen beobachtet, sie tritt nur selten an den Vordergliedmaßen auf. Gelegentlich sind auch im dorsalen Kronsaumbereich und im Zwischenklauenspalt Dermatitis digitalis-typische Läsionen sichtbar. Die Ausdehnung der Hautveränderung kann Durchmesser bis zu 10 cm erreichen. Veränderungen verschiedensten Ausmaßes der Dermatitis digitalis können mit Lahmheit einhergehen. Bei milderen Formen sind die betroffenen Hautbezirke nur druckempfindlich und provozieren beim Tier keine sichtbaren Schmerzäußerungen, werden aber mit gelegentlichem Heben der betroffenen Gliedmaße in Beziehung gebracht.

### Dermatitis interdigitalis (Klauenfäule)

Klauenfäule oder Dermatitis interdigitalis ist eine bei Rindern und anderen Paarhufern, wie beispielsweise Schafen, auftretende Erkrankung der Zwischenklauenhaut. Es handelt es sich um eine multifaktorielle Erkrankung mit infektiösem Hintergrund.

Symptome sind u.a. eine gerötete bis mazerierte Zwischenklauenhaut mit einem feuchten, stinkenden Belag. Meist sind alle vier Gliedmaßen betroffen. In ersten Stadium der Erkrankung verursachen Bakterien eine seröse Entzündung der Haut des Zwischenklauenspalts. Es entsteht ein schmieriger Belag mit käsig-fauligem Geruch. Zunächst ist nur die Oberfläche der Zwischenklauenhaut betroffen, die Veränderungen können jedoch rasch fortschreiten und in die Tiefe eindringen.

### Ekzeme

Als Ekzeme werden nicht-infektiöse Entzündungsreaktionen der Haut bezeichnet, die durch verschiedene Auslöser hervorgerufen werden können. Unterschiedliche Ekzemformen unterscheiden sich dabei hinsichtlich Ursache, Erkrankungsentstehung und typischem Erkrankungsbild. Ein Grundtyp des Ekzems ist das atopische Ekzem, das auch als atopische Dermatitis bezeichnet wird.

### Atopische Dermatitis

Bei der atopischen Dermatitis handelt es sich um eine allergisch bedingte Hauterkrankung, die durch intensiven Juckreiz gekennzeichnet ist. Die atopische Dermatitis ist z.B. eine häufige Hauterkrankung bei Haushunden. Die Ursache und Entstehung der Erkrankung ist noch nicht vollständig aufgeklärt. Vermutet wird eine genetisch bedingte Überempfindlichkeit gegenüber Fremdstoffen. Die Erkrankung wird über eine allergische Reaktion vom Sofort-Typ ausgelöst, die durch Immunglobulin E vermittelt wird. Es wird angenommen, dass darüber hinaus auch andere immunologische Vorgänge mit Beteiligung von Immunglobulin G, Langerhans-Zellen, T-Lymphozyten und eosinophilen Granulozyten beteiligt sind. Das wichtigste und anfangs auch einzige Symptom der atopischen Dermatitis ist Juckreiz. Primäre Hautveränderungen können in Form von Rötungen auftreten. Die häufigsten Lokalisationen der atopischen Dermatitis beim Hund sind Kopf (Lefzen, Augenumgebung, Ohr) und/oder Pfoten. Weiterhin können die Beugeseiten des Ellbogens, des Vordermittelfußes und des Sprunggelenks, die Achselgegend, die Leistenregion und der Bauch betroffen sein.

Im weiteren Verlauf kommt es zu sekundären Hautveränderungen durch das Kratzen, Scheuern, Belecken oder Benagen der juckenden Hautpartien. Hierbei können z.B. oberflächliche Hautdefekte, Knötchen, Eiterbläschen und starke Schuppenbildung, bei längerem Bestehen auch Haarausfall und Hautverdickung auftreten. Folgeerkrankungen sind beispielsweise eine eitrige Hautentzündung (Pyodermie) durch bakterielle Sekundärinfektion.

### Allergiebedingte Hauterkrankungen

Weitere allergiebedingte Hauterkrankungen werden bei Tieren beispielsweise durch Flohbisse und Futtermittelüberreaktionen ausgelöst. Die Flohbissallergie ist die häufigste Hauterkrankung bei der Katze und kann dabei alle gängigen Hautreaktionsmuster hervorrufen. Beim Hund manifestiert sich die Flohbissallergie typischerweise als Juckreiz, Papeln und Krusten an der Schwanzbasis und/oder in der Inguinal- sowie der Umbilikalgegend. Bei schweren Fällen können der gesamte Rumpf sowie die Oberschenkel betroffen sein.

### Erkrankungen der Zitzen- und Euterhaut

Weitere verbreitete Hauterkrankungen bei Säugetieren sind Erkrankungen der Zitzen- und Euterhaut. Derartige Erkrankungen können infektiöse und nichtinfektiöse Ursachen haben und spielen insbesondere bei Milchkühen eine große Rolle.

Zu den bakteriellen Erkrankungen der Zitzen- und Euterhaut, z.B. beim Rind, zählen u.a. Akne, Furunkulose, Abszess, abszedierende Dermatitiden, Aktinobazillose und Nekrose. Nichtinfektiöse Erkrankungen der Zitzen- und Euterhaut umfassen u.a. allergisch bzw. toxisch bedingte Affektionen. Zu dieser Gruppe von Erkrankungen zählen das Nesselfieber (Urtikaria), Kriebelmückenstiche, Arzneimittelallergien, sowie die Milchallergie. Nichtinfektiöse Erkrankungen können auch durch chemische, thermische und mechanische Reize verursacht werden, z.B. Verätzungen, Verbrennungen, Erfrierungen, und Verletzungen an Euter und Zitzen.

Neben Rindern sind auch Haustiere, wie z.B. Hunde und Katzen, häufig von Erkrankungen der Zitzen- und Euterhaut betroffen.

### Euter-Schenkel-Dermatitis

Eine weitere, insbesondere bei Rindern auftretende entzündliche Hauterkrankung ist die Euter-Schenkel-Dermatitis (Euter-Schenkel-Ekzem). Hierbei handelt es sich um eine Hauterkrankung, die z.B. bei Milchkühen auftritt, und von der hauptsächlich Erstkalbende in der Zeit um die Kalbung betroffen sind. Dabei kommt es im intertriginösen Raum zwischen Euter und Schenkelinnenflächen durch verminderte Schweißabdunstung zur Störung des Säuremantels (steigender pH-Wert), Sekretstau, Hautmazeration und schließlich zu einer bakteriellen Infektion.

### 3. Schaumformulierungen

Als eine besondere Anwendungsform kosmetischer und/oder dermatologischer Emulsionen zur Pflege und/oder Behandlung der menschlichen Haut sind Schaumformulierungen bekannt.

Schaumformulierungen haben den Vorteil, sich leicht auf der Haut verteilen zu lassen. Die schaumige Konsistenz wird als angenehm empfunden und die Produkte hinterlassen in der Regel ein gutes Hautgefühl. Insbesondere wirkt sich aber auch die physikalische Struktur des Schaumes positiv auf die Hautschutzfunktion aus. Schäume sind komplizierte physikalische Gebilde, die einer besonderen Abstimmung der den Schaum bildenden Komponenten bedürfen. Schäume werden generell durch Versprühen einer Emulsionsformulierung oder einer wässrigen Tensid-(Stabilisator-)lösung erhalten. Beispielsweise wird die mit Treibgas versetzte Emulsion aus einem unter Druck stehenden Behältnis abgegeben (solche Systeme werden in der Literatur und Patentliteratur auch als Aerosolschäume bezeichnet). Dabei expandiert das unter Druck stehende Gemisch aus Emulsion und Treibgas und bildet die Schaumbläschen. Insbesondere kommt es zu einer Expansion der dispersen Ölphase, in welcher das öllösliche Gas gelöst ist. Schäume können aber auch mit Hilfe anderer Systeme erzeugt werden, wie beispielsweise Pumpsprays.

Abgestimmte Schaumformulierungen weisen eine stabile zwei- oder mehrphasige polydisperse Struktur bei der Applikation auf, die auf der Haut eine Netzstruktur, vergleichbar mit einer Membran, bildet. Solche Netzstrukturen haben den Vorteil, dass diese eine Schutzfunktion, beispielsweise vor Kontakt mit Wasser ausbilden, jedoch einen ungehinderten Gasaustausch mit der Umgebung zulassen. Bei solchen Schäumen kommt es praktisch zu keiner Behinderung der *Perspiratio insensibiles* und keinem entsprechenden Wärmestau. Damit werden die positiven Eigenschaften einer Schutz- und Pflegefunktion mit einer unveränderten Hautatmung verbunden.

In der WO 2007/036376 A2 werden Schaumhautcremes basierend auf einer wässrigen Emulsion, die Harnstoff und Hyaluronsäure enthält, beschrieben, sowie deren Verwendung zur Behandlung von Dermatosen.

Die WO 2010/000348 A2 offenbart Schaumformulierungen basierend auf einer Emulsion, die Mikrosilber enthält, sowie deren Verwendung in der Prophylaxe und/oder Therapie von z.B. Mykosen, Fußpilz, Neurodermitis, und zur Behandlung der Füße bei Diabetes.

Die kosmetische oder dermatologische Anwendung solcher Schaumformulierungen zur Vorbeugung und/oder Behandlung von Hauterkrankungen bei nicht-menschlichen Säugetieren wird in keinem der oben genannten Dokumente beschrieben.

Die internationale Anmeldung WO 2008/075207 offenbart schäumbare Zusammensetzungen, die auf die Körperoberfläche von Säugetieren aufgebracht werden sollen, und einen schäumbaren Träger, eine wirksame Konzentration eines Antiinfektivums, ein "Verstärkungsmittel" (augmenting agent) in Form eines Keratolytikums oder eines Hautpenetrationsverstärkers, und ein Treibmittel enthalten. Als ein bevorzugtes Keratolytikum wird Harnstoff offenbart. Hinsichtlich des in der Zusammensetzung enthaltenen Antiinfektivums wird u.a. die Verwendung von antimikrobiellem Silber als fungizides oder antibiotisches Mittel vorgeschlagen.

Die internationale Anmeldung WO 2010/000348 offenbart eine Formulierung für eine Schaumhautcreme umfassend eine wässrige Emulsion und ein Treibmittel, die in einer Ausführungsform nebst Mikrosilber, Panthenol und Madecassosiden auch Urea enthält. Urea soll dabei eine Erhöhung der Wasserbindungsfähigkeit der Hornschicht bewirken und dadurch die Wirkung der übrigen Bestandteile verbessern.

Die internationale Anmeldung WO 2009/036714 offenbart eine Zusammensetzung für Wundbehandlungen, die Hyaluronsäure, Harnstoff und kolloidales Silber enthält. Es wird außerdem beschrieben, dass die Zusammensetzung als Spray, Salbe, Gel, Suspension, Pulver, Waschlotion, Tinktur, Gaze, Wundauflage, getränkte Wundauflage, Schaum oder Schwamm vorliegen kann.

Die internationale Anmeldung WO 2008/155389 offenbart Schaumformulierungen, umfassend eine Emulsion, umfassend eine Ölphase und eine Wasserphase, wobei die Ölphase mindestens eine membranbildende Substanz umfasst, die in der Schaumformulierung eine lamellar angeordnete Membran ausbildet. Die Schaumformulierungen werden für kosmetische und dermatologische Zwecke vorgeschlagen. Außerdem wird erwähnt, dass DMS®-Cremezusammensetzungen insbesondere bei gereizter, trockener bis sehr trockener, sensitiver bis sehr sensitiver, allergischer und ekzemischer Haut eingesetzt werden. Als optional enthaltener Wirkstoff wird u.a. Harnstoff aufgeführt.

Die Anmeldung US 2009/0041680 offenbart eine schäumbare Zusammensetzung, die u.a. ein flüssiges Wachs umfassend mindestens eine Fettsäure oder mindestens einen Fettalkohol, Wasser und Treibgas enthält, und die durch Auswahl eines geeigneten Wirkstoffes zur Behandlung dermatologischer Erkrankungen bei menschlichen oder tierischen Patienten verwendet werden soll. Es wird beispielhaft vorgeschlagen, den beschriebenen Zusammensetzungen Harnstoff in Konzentrationen von 5-10% zuzusetzen, um Hauterkrankungen wie atopische Dermatitis und Ekzeme zu behandeln.

Die Anmeldung US 2004/0156874 offenbart ein Verfahren zur Behandlung topischer, entzündlicher Hauterkrankungen, bei dem das betroffene Hautareal eines menschlichen oder tierischen Patienten mit einer Zusammensetzung behandelt wird, die aus einer antientzündlich wirksamen Menge Harnstoff und weiteren dermatologisch verträglichen Hilfsstoffen besteht. Es wird außerdem beschrieben, dass die Zusammensetzung u.a. als Creme, Lotion, Lösung, Gel, Lack, Salbe, oder Schaum vorliegen kann.

Die internationale Anmeldung WO 99/08649 offenbart Schaum-Hautcremes enthaltend eine Wasser- und eine Ölphase, die durch ein spezielles Herstellungsverfahren erhältlich sind und sich zur Behandlung oder Linderung von dermatologischen Erkrankungen wie allergisches Kontaktekzem vom Typ I und IV, kumulativ-subtoxisches Ekzem, toxisch-irntatives Ekzem, mikrobiell-dysregulatives Ekzem, atopische Dermatitis, etc. eignen. Es wird offenbart, dass die wässrige Phase der Schaumhautcremes zusätzliche hydratisierende Stoffe, vorzugsweise 1-20 Gew.-% Harnstoff, enthalten kann.

Die internationale Anmeldung WO 2005/016329 offenbart pharmazeutische, kosmetische oder kosmezeutische Zusammensetzungen zur topischen Anwendung, die als Wirkstoffe Harnstoff und Ammoniumlactat enthalten, und zur Behandlung von Hauterkrankungen wie Akne, Dermatitis, Neurodermatitis, Ekzem, atopisches Ekzem, etc. vorgeschlagen werden. Die Zusammensetzungen können als Creme, Salbe, Paste, Gel, Lotion, Milch, Suspension, Aerosol, Spray, Schaum, Shampoo, Haar-Conditioner, Serum, Tupfer, etc. vorliegen.

Die Anmeldung EP 1 287 814 offenbart ein äusserlich anzuwendendes Mittel zur Förderung der Abwehrleistung der Haut gegenüber chemischen und physikalischen Irritationen, enthaltend ionisch gebundene und/oder freie Thiocyanationen und Harnstoff neben an sich bekannten Hilfs- und Trägerstoffen, dadurch gekennzeichnet, dass das Gewichtsverhältnis von Thiocyanationen zu Harnstoff und/oder Harnstoffderivaten im Bereich von 10 : 1 bis 250 : 1 liegt. Das Mittel kann Salben, Cremes, Emulsionen, Lotionen und/oder alkoholische wässrige Lösungen umfassen.

### Zusammenfassung der Erfindung

Die Anmelderin hat nun erkannt, dass sich sowohl Mikrosilber, als auch Harnstoff, zur topischen Behandlung der Haut nicht-menschlicher Säugetiere, insbesondere erkrankter oder erkrankungsgefährdeter Haut, und zu deren kosmetischer Pflege eignen. Insbesondere hat die Anmelderin erkannt, dass Schaumformulierungen umfassend eine Emulsion, wobei die Emulsion Mikrosilber und/oder Harnstoff umfasst, zur Vorbeugung und/oder Behandlung von Hauterkrankungen bei nicht-menschlichen Säugetieren besonders geeignet sind, wobei die Hauterkrankungen ausgewählt sind aus der Gruppe bestehend aus Mauke, Raspe oder ähnlichen Hauterkrankungen.

Dabei können die Schaumformulierungen zusätzlich zu den oben beschriebenen positiven Eigenschaften weitere vorteilhafte Wirkungen ausüben. Die erfindungsgemäß verwendeten Schaumformulierungen lassen sich selbst auf stark behaarte, tierische Haut gut auftragen und ziehen rasch ein, ohne fettende Substanzen auf Haut und Fell zurückzulassen. Ein Verkleben von behaarten Hautarealen wird dadurch vermieden.

Dies ist insbesondere von Bedeutung in der Behandlung von Hauterkrankungen an Körperpartien, die vermehrt mit Schmutz in Berührung kommen, wie beispielsweise der Fesselbereich bei Pferden oder Rindern. An solchen Hautpartien tragen stärker fettende Produkte, wie etwa Salben oder Cremes, dazu bei, dass vermehrt Schmutzpartikel oder Fremdkörper (z.B. Einstreu) anhaften und so die Abheilung von Hautläsionen erschwert wird. Dies kann durch die Verwendung der erfindungsgemäßen Schaumformulierungen vermieden werden.

Ein weiterer Vorteil ist, dass die erfindungsgemäß verwendeten Harnstoff- und/oder Mikrosilber-haltigen Schaumformulierungen die Behandlung von Hauterkrankungen nicht-menschlicher Säugetiere ohne den Einsatz von z.B. dopingrelevanten Wirkstoffen ermöglichen. Ein therapeutischer oder metaphylaktischer Einsatz ist somit, beispielsweise bei Sportpferden, auch in der Turniersaison möglich.

Ein weiterer, besonderer Vorteil der Verwendung von Mikrosilber ist, dass dieses, im Vergleich zu beispielsweise nanokristallinen Silberpartikeln, aufgrund seiner mittleren Teilchengröße von typischerweise etwa 10 µm die Haut von nicht-menschlichen Säugetieren nicht penetriert. Möglicherweise toxische Wirkungen können dadurch vermieden werden. Ferner erlaubt Mikrosilber aufgrund seiner physikalischen Struktur eine kontinuierliche Generierung von antimikrobiell wirkenden Silberionen über einen längeren Zeitraum und hat dadurch einen Depoteffekt.

Die Erfindung betrifft somit eine Schaumformulierung umfassend eine Emulsion, umfassend eine Ölphase und eine Wasserphase, wobei die Emulsion Mikrosilber und/oder Harnstoff umfasst, zur Verwendung in der Vorbeugung und/oder Behandlung einer Hauterkrankung bei nicht-menschlichen Säugetieren, wobei die Hauterkrankung ausgewählt ist aus der Gruppe bestehend aus Mauke, Raspe oder ähnlichen Hauterkrankungen. Ebenso betrifft die Erfindung die Verwendung einer derartigen Schaumformulierung zur Herstellung eines topischen Medikaments zur Vorbeugung und/oder Behandlung einer der soeben genannten Hauterkrankung bei nicht-menschlichen Säugetieren.

Ferner betrifft die Erfindung Harnstoff zur Verwendung in der Vorbeugung und/oder Behandlung einer Hauterkrankung bei nicht-menschlichen Säugetieren, wobei die Hauterkrankung ausgewählt ist aus der Gruppe bestehend aus Mauke, Raspe oder ähnlichen Hauterkrankungen. Ebenso betrifft die Erfindung die Verwendung von Harnstoff zur Herstellung eines topischen Medikaments zur Vorbeugung und/oder Behandlung der soeben genannten Hauterkrankungen bei nicht-menschlichen Säugetieren.

Außerdem betrifft die Erfindung Mikrosilber zur Verwendung in der Vorbeugung und/oder Behandlung einer Hauterkrankung bei nicht-menschlichen Säugetieren, wobei die Hauterkrankung ausgewählt ist aus der Gruppe bestehend aus Mauke, Raspe, oder ähnlichen Hauterkrankungen. Ebenso betrifft die Erfindung die Verwendung von Mikrosilber zur Herstellung eines topischen Medikaments zur Vorbeugung und/oder Behandlung einer der soeben genannten Hauterkrankungen bei nicht-menschlichen Säugetieren.

Weiterhin betrifft die Erfindung eine harnstoffhaltige wässrige Lösung zur Verwendung in der Entfernung von Krusten auf erkrankten Hautarealen von an Mauke, Raspe oder ähnlichen Hauterkrankungen erkrankten nicht-menschlichen Säugetieren, sowie die Verwendung einer harnstoffhaltigen wässrigen Lösung als Kosmetikum oder zur Herstellung eines topischen Medikaments zu dem genannten Zweck.

Schließlich betrifft die Erfindung einen Kit umfassend eine harnstoffhaltige wässrige Lösung und eine Schaumformulierung umfassend eine Emulsion umfassend eine Ölphase und eine Wasserphase, wobei die Emulsion Mikrosilber und/oder Harnstoff umfasst.

Gemäß einem weiteren Aspekt betrifft die vorliegende Erfindung auch die Verwendung von Harnstoff und/oder Mikrosilber, insbesondere von Schaumformulierungen umfassend eine Emulsion, umfassend eine Ölphase und eine Wasserphase, wobei die Emulsion Mikrosilber und/oder Harnstoff umfasst, zur kosmetischen Pflege der Haut nicht-menschlicher Säugetiere bei einer drohenden Hauterkrankung, wobei die Hauterkrankung ausgewählt ist aus der Gruppe bestehend aus Mauke, Raspe oder ähnlichen Hauterkrankungen.

Die Eignung zur Verwendung auch in der kosmetischen Pflege ergibt sich dabei insbesondere aus den positiven Auswirkungen der Mikrosilber und/oder Harnstoff enthaltenden Darreichungsformen, insbesondere Schaumformulierungen, auf die Haut nicht-menschlicher Säugetiere, wie z.B. Erhöhung des Feuchtigkeitsgehalts der Haut, oder Verbesserung der Hautelastizität und -geschmeidigkeit und damit verbunden Erhöhung der Widerstandskraft der Haut.

### Detaillierte Beschreibung der Erfindung

### 1. Definitionen

Gemäß der vorliegenden Erfindung sind Schaumformulierungen Formulierungen, insbesondere Emulsionen, die zur Erzeugung von Schaum sinnfällig hergerichtet sind. Die Formulierungen können insbesondere entweder mit (druck)verflüssigtem Treibgas in einen Druckbehälter abgefüllt sein oder ohne Treibgas in einen anderen Behälter als einen Druckbehälter abgefüllt sein, der es ermöglicht, bei Abgabe der Formulierung/Emulsion einen Schaum zu erzeugen. Beispielsweise können Pumpspraybehälter verwendet werden.

Gemäß der vorliegenden Erfindung sind Sprayformulierungen Formulierungen, insbesondere wässrige Lösungen, die zur Anwendung durch Versprühen sinnfällig hergerichtet sind. Die Formulierungen können insbesondere entweder mit (druck)verflüssigtem Treibgas in einen Druckbehälter abgefüllt sein oder ohne Treibgas in einen anderen Behälter als einen Druckbehälter abgefüllt sein, der es ermöglicht, die Formulierung zu versprühen. Beispielsweise können Pumpspraybehälter verwendet werden.

Gemäß der vorliegenden Erfindung sind "ähnliche Hauterkrankungen" Hauterkrankungen, die eine gleiche oder ähnliche Pathogenese und Ätiologie aufweisen, wie die Bezug genommene Hauterkrankung. Typischerweise äußern sich "ähnliche Hauterkrankungen" auch in ähnlichen Symptomen. Eine "ähnliche Hauterkrankung" ist beispielsweise eine Hauterkrankung gleicher oder ähnlicher Symptomatik und Pathogenese bei einem anderen Tier.

### 2. Zusammensetzung der topischen Darreichungsform

Die erfindungsgemäß verwendeten Wirkstoffe Mikrosilber und/oder Harnstoff werden vorzugsweise in Form einer Emulsion, einer Lotion, eines Gels, einer Creme, einer Salbe, eines Puders, einer Suspension, einer Lösung, eines Shampoos, einer Tinktur oder einer Schaumformulierung verwendet. Bevorzugt sind dabei eine Emulsion oder eine Schaumformulierung umfassend eine Emulsion, besonders bevorzugt eine Schaumformulierung umfassend eine Emulsion.

Erfindungsgemäß verwendbare Schaumformulierungen, die als Träger für die Wirkstoffe Mikrosilber und/oder Harnstoff, sowie optional weiterer hierin beschriebener Wirkstoffe dienen können, und die für die erfindungsgemäßen Verwendungen geeignet sind, insbesondere die Zusammensetzung der den Schaumformulierungen zu Grunde liegenden Emulsionen, sowie Verfahren zur Herstellung solcher Schaumformulierungen, sind beispielsweise in den Veröffentlichungen WO 99/08649, WO 2004/052317 A1, WO 2007/036376 A2, WO 2008/138894 A2 und WO 2008/155389 A2 beschrieben.

Im Folgenden werden bevorzugte Ausführungsformen der Schaumformulierungen, insbesondere der den Schaumformulierungen zu Grunde liegenden Emulsionen, beschrieben. Wie für den Fachmann ersichtlich, können die bevorzugten Ausführungsformen der Emulsionen jedoch auch als Grundlage für andere Emulsions-basierte topische Darreichungsformen dienen, wie etwa Lotionen oder Cremes.

In einer Ausführungsform umfasst die Emulsion Harnstoff. Vorzugsweise umfasst die Emulsion dabei von 3 bis 30 Gew.-% Harnstoff, besonders bevorzugt von 5 bis 25 Gew.-% Harnstoff, ganz besonders bevorzugt von 5 bis 20 Gew.-% Harnstoff (jeweils bezogen auf das Gesamtgewicht der Emulsion ohne Treibgas).

In einer anderen Ausführungsform umfasst die Emulsion Mikrosilber.

"Mikrosilber" gemäß der vorliegenden Erfindung bezeichnet metallisches Silber enthaltende Partikel (im Folgenden "Silberpartikel" genannt) mit einer mittleren Partikelgröße von 1 µm bis 100 µm und einer spezifischen Oberfläche von 2 m²/g bis 10 m²/g. Ein derartiges Produkt ist beispielsweise unter der Bezeichnung "MicroSilver BG™" von der Firma BioEpiderm GmbH in Nürnberg erhältlich.

Vorzugsweise beträgt die mittlere Partikelgröße der Silberpartikel dabei von 2 µm bis 20 µm, besonders bevorzugt von 2 µm bis 15 µm, ganz besonders bevorzugt ungefähr 10 µm. Ferner bevorzugt kann die spezifische Oberfläche der Silberpartikel von 3 m²/g bis 6 m²/g, insbesondere ungefähr 5 m²/g, betragen. Die spezifische Oberfläche kann z.B. mittels N₂-Adsorption volumetrisch nach dem BET-Verfahren (Brunauer-Emmet-Teller-Verfahren) bestimmt werden.

Derartige Silberpartikel können nach im Stand der Technik bekannten Verfahren hergestellt werden. Dazu zählen beispielsweise das mechanische Zermahlen in Kolloidmühlen sowie die elektrolytische Herstellung über verschiedene Verfahren wie z. B. mit Umkehrosmosewasser oder dampfdestilliertem Wasser in erwärmtem Zustand. Auch rein chemische Verfahren zur Herstellung von Silberpartikeln einer Grösse von 1 µm bis 100 µm über Reduktion von Silbersalzen sind anwendbar.

Die erfindungsgemäßen Silberpartikel enthalten vorzugsweise mindestens 99 Gew.-%, bevorzugt mindestens 99,5 Gew.-% oder 99,9 Gew.-% metallisches Silber. Sie können aber auch in einem geringen Anteil von bis zu 1 Gew.-% andere Metalle enthalten, wie z. B. Zink und/oder Kupfer. Beispielsweise können die Silberpartikel bis zu 0,5 Gew.-% metallisches Zink und/oder bis zu 0,5 Gew.-% metallisches Kupfer enthalten. Zu diesem Zweck können die Partikel z.B. als Silber-ZinkLegierung oder als Silber-Zink-Kupfer-Legierung vorliegen. Besonders vorteilhaft weisen die Silberpartikel Verunreinigungen an Kalium, Natrium oder Chlor von weniger als 5 ppm auf.

Vorzugsweise sind die Silberpartikel porös. Dabei weisen die Silberpartikel vorzugsweise eine mittlere innere Porosität von mindestens 65%, insbesondere von 65 bis 95%, besonders bevorzugt von 85 bis 95% auf. Unter innerer Porosität wird der prozentuale Anteil des Volumens des Partikels verstanden, der nicht von Metall ausgefüllt ist. Die mittlere innere Porosität der Partikel kann dabei nach dem in WO 2005/023213 A1 auf Seite 7 beschriebenen Verfahren bestimmt werden.

Vorzugsweise liegen die Silberpartikel als Agglomerate metallischer Primärpartikel vor. Die Agglomerate können aus Primärpartikeln mit einem mittleren Durchmesser zwischen 10 und 200 nm, vorzugsweise zwischen 15 und 80 nm, gebildet sein. Das Vorliegen derartiger Strukturen kann elektronenmikroskopisch nachgewiesen werden. Zur Herstellung solcher Silberpartikel kann beispielsweise das in WO 2005/023213 A1 auf Seite 10 beschriebene Verfahren verwendet werden.

In einer bevorzugten Ausführungsform weisen die Silberpartikel eine schwammartige Struktur auf. Dies resultiert in einer großen Oberfläche, die sich vorteilhaft auf die Freisetzung von Silberionen auswirkt.

In den Ausführungsformen, in denen die Emulsion Mikrosilber umfasst, ist dieses bevorzugt in einer Menge von 0,01 bis 3 Gew.-%, mehr bevorzugt von 0,01 bis 1,5 Gew.-%, besonders bevorzugt von 0,01 bis 1 Gew.-%, ganz besonders bevorzugt von 0,01 bis 0,5 Gew.-% enthalten (jeweils bezogen auf das Gesamtgewicht der Emulsion ohne Treibgas).

In einer besonders bevorzugten Ausführungsform umfasst die Emulsion neben dem Mikrosilber zusätzlich Harnstoff, vorzugsweise in den oben angegebenen Mengen.

Die Harnstoff und/oder Mikrosilber enthaltenden Emulsionen können vorzugsweise weitere Wirkstoffe oder Substanzen mit z.B. hautberuhigender, regenerierender, wundheilungsfördernder, oder feuchtigkeitsspendender Wirkung enthalten. Geeignete Wirkstoffe sind beispielsweise in den internationalen Anmeldungen WO 2008/138894 A2, Seiten 14 bis 16 und WO 2008/155389 A2, Seiten 26 bis 27 beschrieben, deren Inhalt hiermit durch Verweis einbezogen wird.

Vorzugsweise umfasst die Emulsion als weiteren Inhaltsstoff Allantoin. Beispielsweise kann die Emulsion von 0,01 bis 5 Gew.-% Allantoin, bevorzugt von 0,01 bis 1 Gew.-% Allantoin, besonders bevorzugt von 0,01 bis 0,5 Gew.-% Allantoin umfassen (jeweils bezogen auf das Gesamtgewicht der Emulsion ohne Treibgas).

In einer bevorzugten Ausführungsform umfasst die Emulsion ferner Nachtkerzenöl. Beispielsweise umfasst die Emulsion von 0,01 bis 8 Gew.-% Nachtkerzenöl, bevorzugt von 0,1 bis 5 Gew.-% Nachtkerzenöl, besonders bevorzugt von 0,5 bis 2,5 Gew.-% Nachtkerzenöl (jeweils bezogen auf das Gesamtgewicht der Emulsion ohne Treibgas).

In einer weiteren bevorzugten Ausführungsform umfasst die Emulsion ferner Sorbitol. Beispielsweise umfasst die Emulsion von 0,01 bis 8 Gew.-% Sorbitol, bevorzugt von 0,1 bis 5 Gew.-% Sorbitol, besonders bevorzugt von 0,5 bis 2,5 Gew.-% Sorbitol (jeweils bezogen auf das Gesamtgewicht der Emulsion ohne Treibgas).

In einer weiteren Ausführungsform umfasst die Emulsion ferner Aloe Vera-Extrakt. Beispielsweise umfasst die Emulsion von 0,01 bis 5 Gew.-% Aloe Vera-Extrakt, bevorzugt von 0,01 bis 3 Gew.-% Aloe Vera-Extrakt, besonders bevorzugt von 0,01 bis 1 Gew.-% Aloe Vera-Extrakt (jeweils bezogen auf das Trockengewicht des Aloe Vera Extrakts und das Gesamtgewicht der Emulsion ohne Treibgas).

In einer weiteren bevorzugten Ausführungsform umfasst die Emulsion ferner Panthenol. Beispielsweise umfasst die Emulsion von 0,2 bis 20 Gew.-% Panthenol, bevorzugt von 0,5 bis 15 Gew.-% Panthenol, besonders bevorzugt von 1 bis 10 Gew.-% Panthenol (jeweils bezogen auf das Gesamtgewicht der Emulsion ohne Treibgas). Von Panthenol existieren zwei Enantiomere, die D- und die L-Form. Nur D-Panthenol ist biologisch aktiv. Erfindungsgemäß wird bevorzugt D-Panthenol verwendet, alternativ kann aber auch L-Panthenol oder ein Gemisch der Enantiomere verwendet werden.

In einer weiteren bevorzugten Ausführungsform umfasst die Emulsion ferner Madecassosid. Madecassosid, auch Gota Kola genannt, ist ein stark konzentrierter Extrakt aus Tigergras ("Centenella Asiatica"), der die Kollagensynthese anregt und als Radikalfänger wirkt und dadurch die Zellerneuerung der Hautoberfläche aktiviert.

Beispielsweise umfasst die Emulsion von 0,01 bis 5 Gew.-%, bevorzugt von 0,01 bis 1 Gew.-%, besonders bevorzugt von 0,01 bis 0,5 Gew.-% Madecassosid (jeweils bezogen auf das Gesamtgewicht der Emulsion ohne Treibgas).

In einer weiteren bevorzugten Ausführungsform umfasst die Emulsion ferner Zinkoxid, bevorzugt in einer Menge von 0,1 Gew.-% bis 10 Gew.-%, mehr bevorzugt von 0,1 Gew.-% bis 5 Gew.-%.

In einer besonders bevorzugten Ausführungsform umfasst die Emulsion ferner mindestens ein Silicon, wie beispielsweise Dimethicone, bevorzugt in einer Menge von 0,1 Gew.-% bis 10 Gew.-%, mehr bevorzugt von 0,1 Gew.-% bis 5 Gew.-%, besonders bevorzugt von 0,1 Gew.-% bis 1 Gew.-%.

In einer bevorzugten Ausführungsform ist die Emulsion eine Öl-in-Wasser Emulsion.

Eine erfindungsgemäß besonders geeignete Schaumformulierung ist eine Schaumcreme.

In einer weiteren bevorzugten Ausführungsform umfasst die Schaumformulierung ein Treibgas. Geeignete Treibgase sind z. B. N₂O, Propan, Butan und i-Butan. Die fertige Schaumformulierung enthält beispielsweise von 1 bis 20 Gew.-%, von 2 bis 18 Gew.-% oder von 5 bis 15 Gew.-%, bevorzugt etwa 10 Gew.-% Treibgas. Bei der Beaufschlagung der Emulsion mit Treibgas wird (druck)verflüssigtes Treibgas verwendet.

### 3. Hauterkrankungen

Bei den Hauterkrankungen handelt es sich vorzugsweise um Hauterkrankungen nicht-menschlicher Säugetiere, die ein Fell aufweisen. Ein Fell gemäß der vorliegenden Erfindung ist eine behaarte Körperhaut mit einer Haardichte von mindestens 500 Haaren pro cm².

In einer weiteren Ausführungsform sind die nicht-menschlichen Säugetiere ausgewählt aus der Gruppe bestehend aus Pferden, Eseln, Mauleseln, Maultieren, Rindern, Schweinen, Schafen, Ziegen, Kamelen, Hunden, Katzen, Hasen, Kaninchen und Meerschweinchen.

Im Folgenden werden bevorzugte, mit den erfindungsgemäß verwendeten Schaumformulierungen behandelbare Hauterkrankungen beschrieben. "Behandelbar" bedeutet in diesem Zusammenhang, dass die Darreichungsform geeignet ist zur Vorbeugung und/oder Behandlung der Hauterkrankung, bzw. zur kosmetischen Pflege von an der Hauterkrankung erkrankter Haut bzw. zu erkranken drohender Haut.

Es hat sich überraschenderweise gezeigt, dass harnstoffhaltige Schaumformulierungen zur Vorbeugung und/oder Behandlung von, bzw. zur kosmetischen Pflege bei, Mauke, Raspe oder ähnlichen Hauterkrankungen, bevorzugt bei Pferden, Eseln, Mauleseln, oder Maultieren, besonders bevorzugt bei Pferden, geeignet sind.

Die mit harnstoffhaltigen Schaumformulierungen behandelbaren Hauterkrankungen sind daher ausgewählt aus der Gruppe bestehend aus Mauke, Raspe, oder ähnlichen Hauterkrankungen.

Enthält die den Schaumformulierungen zu Grunde liegende Emulsion Mikrosilber, vorzugsweise in Kombination mit Harnstoff, so umfassen die behandelbaren Hauterkrankungen ebenfalls Mauke, Raspe oder ähnliche Hauterkrankungen

Die Mikrosilber-haltigen Schaumformulierungen eignen sich besonders zur Vorbeugung und/oder Behandlung von Mauke, Raspe oder ähnlichen Hauterkrankungen bei Pferden, Eseln, Mauleseln, oder Maultieren, besonders bevorzugt bei Pferden.

Wie für den Fachmann ersichtlich, sind die oben in Bezug auf Harnstoff- bzw. Mikrosilber-haltige Schaumformulierungen genannten Hauterkrankungen auch mit anderen Harnstoff- bzw. Mikrosilber-haltigen topischen Darreichungsformen, wie etwa Emulsionen, behandelbar. Analoges gilt für die im Folgenden beschriebene Behandlung der Hauterkrankungen.

### 4. Behandlung

Die Behandlung oder Pflege der erkrankten oder erkrankungsgefährdeten Haut umfasst das topische Auftragen der erfindungsgemäß verwendeten Schaumformulierungen auf die betroffenen Hautareale. Das Auftragen kann beispielsweise einmal täglich erfolgen. Je nach Schweregrad der Erkrankung ist auch ein zweimal oder mehrmal tägliches Auftragen der Schaumformulierungen möglich, oder ein Auftragen der Schaumformulierungen an jedem zweiten oder dritten Tag. Insbesondere zur Vorbeugung der Hauterkrankungen bzw. zur kosmetischen Pflege der Haut können die erfindungsgemäß verwendeten Schaumformulierungen z.B. einmal täglich, bzw. ein-, zwei oder dreimal wöchentlich auf gefährdete Hautareale aufgetragen werden.

Die erfindungsgemäß verwendeten Schaumformulierungen können auch mit dermatologischen Arzneimitteln, z.B. topisch oder oral zu verabreichenden Arzneimitteln, kombiniert werden. Werden die erfindungsgemäß verwendeten Schaumformulierungen in Kombination mit einem oder mehreren weiteren topisch anzuwendenden Arzneimitteln eingesetzt, erfolgt das Auftragen der erfindungsgemäß verwendeten Schaumformulierungen und der weiteren topisch anzuwendenden Arzneimittel vorzugsweise nicht gleichzeitig, sondern zeitversetzt. Es hat sich gezeigt, dass bei einer topischen Behandlung erkrankter Hautareale mit den erfindungsgemäß verwendeten Schaumformulierungen z.B. die Dauer einer parallelen Antibiotika-Behandlung verkürzt werden konnte.

In einer bevorzugten Ausführungsform wird das betroffene Hautareal sowie gegebenenfalls umgebende Hautareale vor dem topischen Auftragen der erfindungsgemäß verwendeten Schaumformulierungen gereinigt, z.B. gewaschen oder gespült. Beispielsweise kann die Reinigung einmalig vor dem erstmaligen topischen Auftragen der erfindungsgemäß verwendeten Schaumformulierungen erfolgen.

Das Waschen kann dabei unter Verwendung eines Shampoos, vorzugsweise eines antibakteriellen Shampoos, oder einer milden Seife, wie Iod-, Schmier- oder Kernseife, erfolgen. Spülungen erfolgen beispielsweise unter Verwendung von Iod-Lösung (Polyvidon-Iod), oder dreiprozentiger wässriger Wasserstoffperoxid-Lösung.

Bei der Behandlung von oder kosmetischen Pflege bei Mauke kann beispielsweise vor dem Auftragen der erfindungsgemäß verwendeten Schaumformulierungen eine Reinigung der erkrankten und umgebender Hautareale mit z.B. Iodseife, Schmierseife, Kernseife, Iod-Lösung (Polyvidon-Iod), oder dreiprozentiger wässriger Wasserstoffperoxid-Lösung erfolgen.

Einige Hauterkrankungen beim Tier, wie z.B. die Mauke beim Pferd, gehen mit der Ausbildung von zum Teil großflächigen Krusten auf den erkrankten Hautarealen einher. Derartige Krusten können neben Entzündungssekreten abgestorbene Epithelzellen, Haare, Schmutzpartikel, Blut und Bakterien umfassen. Insbesondere unter großflächigeren Krusten können sich vorhandene Bakterien, Pilze, aber auch Parasiten ungestört vermehren und das Krankheitsbild weiter verschlimmern.

Vorzugsweise werden entstandene Krusten deshalb vor dem Auftragen der erfindungsgemäß verwendeten Schaumformulierungen entfernt, insbesondere bei der Behandlung der Mauke. Da vor allem bei starken Verkrustungen die Entfernung der Krusten schmerzhaft sein kann, und um zusätzliche Hautverletzungen zu vermeiden, können die Krusten zunächst aufgeweicht oder aufgelöst werden. Das Aufweichen oder Auflösen kann beispielsweise durch Anwendung von nassen Verbänden, wie z.B. Rivanol-Verbänden, erfolgen. In einer bevorzugten Ausführungsform erfolgt das Aufweichen oder Auflösen der Krusten, z.B. bei Mauke-Patienten, mit keratolytisch wirksamen Präparaten (z.B. Tinkturen, Salben, Cremes oder Lotionen enthaltend keratolytisch wirksame Substanzen, etwa Harnstoff, Salicylsäure, oder Hydroxycarbonsäuren).

In einer besonders bevorzugten Ausführungsform wird für das zumindest teilweise Aufweichen oder Auflösen der Krusten, z. B. bei Mauke-Patienten, eine Behandlung mit einer harnstoffhaltigen wässrigen Lösung durchgeführt.

Gemäß einer bevorzugten Ausführungsform der Erfindung umfasst die Behandlung von, bzw. die kosmetische Pflege bei, Mauke, Raspe oder ähnlichen Hauterkrankungen somit folgende Schritte:
(a) Behandeln von Krusten auf erkrankten Hautarealen mit einer keratolytisch wirksamen Substanz, vorzugsweise mit einer harnstoffhaltigen wässrigen Lösung wie hierin beschrieben;
(b) Entfernen der in Schritt (a) behandelten Krusten;
(c) topisches Auftragen von Mikrosilber, vorzugsweise einer Mikrosilber-haltigen Schaumformulierung wie hierin beschrieben, auf die erkrankten Hautareale, oder topisches Auftragen von Harnstoff, vorzugsweise einer harnstoffhaltigen Schaumformulierung wie hierin beschrieben, auf die erkrankten Hautareale.

Zu diesem Zweck kann vorteilhaft ein Kit verwendet werden, umfassend:
a) eine harnstoffhaltige wässrige Lösung wie hierin beschrieben, und
b) eine Schaumformulierung umfassend eine Emulsion wie hierin beschrieben, wobei die Emulsion Mikrosilber und/oder Harnstoff umfasst.

Ein Kit gemäß der vorliegenden Erfindung schließt bevorzugt die Verpackung und gedruckte Anweisungen für seine Verwendung, z.B. auf einer Packungsbeilage, ein. Diese Anweisungen umfassen dabei vorzugsweise die soeben wiedergegebenen Behandlungsschritte a) bis c). Weiterhin kann der Kit die Reihenfolge der Verwendung der enthaltenen Darreichungsformen kennzeichnen, z.B. durch Nummerierung.

Gegenüber harnstoffhaltigen Cremes, bei denen die Konzentration an Harnstoff meist zwischen 3 und 10 Gew.-% bezogen auf das Gesamtgewicht der Creme beträgt, können in der erfindungsgemäß verwendeten, wässrigen Lösung höhere Konzentrationen an Harnstoff eingesetzt werden. Dadurch wird das Aufweichen oder Auflösen der Krusten verbessert.

Die erfindungsgemäß verwendete harnstoffhaltige wässrige Lösung enthält bevorzugt von 5 bis 35 Gew.-%, besonders bevorzugt von 12 bis 30 Gew.-%, ganz besonders bevorzugt von 15 bis 20 Gew.-% Harnstoff (jeweils bezogen auf das Gesamtgewicht der wässrigen Lösung). Beispielsweise kann die wässrige Lösung ungefähr 18 Gew.-% Harnstoff enthalten.

Die harnstoffhaltige wässrige Lösung kann neben Harnstoff auch weitere kosmetische oder pharmazeutische Wirkstoffe enthalten. Der gegebenenfalls enthaltene weitere Wirkstoff kann dabei unter allen oberflächig auf der Haut applizierbaren Wirkstoffen und Mischungen dieser gewählt werden.

Geeignete Wirkstoffe umfassen beispielsweise Substanzen mit feuchtigkeitspendenden und barrierestärkenden Eigenschaften, wie z. B. Hydroviton, Pyrrolidoncarbonsäure und deren Salze, Milchsäure und deren Salze, Glycerol, Sorbitol, Propylenglykol, oder Substanzen mit hautberuhigender und regenerierender Wirkung, wie z. B. Allantoin oder Panthenol.

Ferner kann die erfindungsgemäß verwendete harnstoffhaltige wässrige Lösung weitere keratolytisch wirkende Substanzen enthalten. Geeignete Keratolytika sind beispielsweise Allantoin, Hydroxycarbonsäuren, z.B. Milchsäure, Mandelsäure und Glycolsäure, Thioglycolsäure, Salicylsäure oder Resorcin.

In einer bevorzugten Ausführungsform enthält die harnstoffhaltige wässrige Lösung Allantoin, vorzugsweise in einer Menge von 0,1 bis 5 Gew.-%. In einer anderen bevorzugten Ausführungsform enthält die harnstoffhaltige wässrige Lösung Panthenol, vorzugsweise in einer Menge von 0,1 bis 5 Gew.-%. In einer weiteren bevorzugten Ausführungsform enthält die harnstoffhaltige wässrige Lösung Allantoin und Panthenol, vorzugsweise jeweils in einer Menge von 0,1 bis 5 Gew.-%. Die Angaben beziehen sich dabei jeweils auf das Gesamtgewicht der wässrigen Lösung.

In einer weiteren bevorzugten Ausführungsform enthält die harnstoffhaltige wässrige Lösung von 0,1 bis 5 Gew.-% Milchsäure, mehr bevorzugt von 0,1 bis 1 Gew.-%, Milchsäure (jeweils bezogen auf das Gesamtgewicht der wässrigen Lösung).

In einer weiteren bevorzugten Ausführungsform enthält die harnstoffhaltige wässrige Lösung ferner ein Alkali- oder Erdalkalimetallhydroxid, wie Kaliumhydroxid, Natriumhydroxid, oder Calciumhydroxid, vorzugsweise in einer Menge von 0,01 bis 0,5 Gew.-%, mehr bevorzugt von 0,01 bis 0,25 Gew.-%, (jeweils bezogen auf das Gesamtgewicht der wässrigen Lösung). Besonders bevorzugt ist dabei Natriumhydroxid.

In einer weiteren Ausführungsform kann die harnstoffhaltige wässrige Lösung ferner Konservierungsmittel enthalten, vorzugsweise in einer Menge von 0,1 bis 5 Gew.-% (bezogen auf das Gesamtgewicht der wässrigen Lösung). Geeignete Konservierungsmittel umfassen Phenoxyethanol, Parabene, Natriumbezoat, Benzoesäure und Benzylalkohol.

Vorzugsweise wird die harnstoffhaltige wässrige Lösung mit einer Sprühvorrichtung enthaltend die harnstoffhaltige wässrige Lösung appliziert. Dabei kann die Sprühvorrichtung Treibmittel verwenden. In diesem Fall liegt die harnstoffhaltige wässrige Lösung in Form einer Sprayformulierung vor, die zusätzlich ein (druck)verflüssigtes Treibgas enthält. Geeignete Treibgase sind z. B. N₂O, Propan, Butan und i-Butan. Es kann sich bei der Sprühvorrichtung aber auch um ein Pumpspray handeln.

Die Verwendung einer Sprühvorrichtung zum Aufbringen der harnstoffhaltigen wässrigen Lösung erlaubt eine einfache Dosierung, konstante Konzentration und insbesondere eine hygienische Anwendung, da ein Kontakt zwischen der erkrankten Haut und der Lösung vermieden wird. Darüber hinaus kann die Anwendung "einhändig" erfolgen, so dass die zweite Hand der behandelnden Person für andere Aufgaben freibleibt.

Insbesondere gestattet die harnstoffhaltige wässrige Lösung ein schonendes Auflösen der Krusten auf der erkrankten Haut. Durch die Verwendung des feuchtigkeitsspendenden Harnstoffs, insbesondere in Kombination mit weiteren feuchtigkeitsspendenden und/oder hautberuhigenden Inhaltsstoffen wie z.B. Milchsäure, Allantoin und Panthenol können zusätzliche Irritationen der erkrankten Hautareale weitgehend vermieden werden.

Durch das Auflösen der Krusten und das damit einhergehende Aufweichen möglicherweise verhornter Hautpartien sind die erkrankten Hautareale nach der Vorbehandlung mit der harnstoffhaltigen wässrigen Lösung besonders aufnahmefähig für die im Anschluss aufgetragenen erfindungsgemäß verwendeten Schaumformulierungen.

### 5. Besondere Ausführungsformen

Die vorliegende Erfindung ist insbesondere auf die folgenden, bevorzugten und nummerierten Ausführungsformen gerichtet:
1. Schaumformulierung umfassend eine Emulsion, umfassend eine Ölphase und eine Wasserphase, wobei die Emulsion Mikrosilber und/oder Harnstoff umfasst, zur Verwendung in der Vorbeugung und/oder Behandlung einer Hauterkrankung bei nicht-menschlichen Säugetieren, wobei die Hauterkrankung ausgewählt ist aus der Gruppe bestehend aus Mauke, Raspe oder ähnlichen Hauterkrankungen.
2. Verwendung einer Schaumformulierung umfassend eine Emulsion, umfassend eine Ölphase und eine Wasserphase, wobei die Emulsion Mikrosilber und/oder Harnstoff umfasst,
   a) zur Herstellung eines topischen Medikaments zur Vorbeugung und/oder Behandlung einer Hauterkrankung bei nicht-menschlichen Säugetieren, oder
   b) zur kosmetischen Pflege der Haut nicht-menschlicher Säugetiere bei einer drohenden Hauterkrankung, wobei die Hauterkrankung ausgewählt ist aus der Gruppe bestehend aus Mauke, Raspe oder ähnlichen Hauterkrankungen.
3. Schaumformulierung zur Verwendung oder Verwendung gemäß Ausführungsform 1 oder 2, wobei die nicht-menschlichen Säugetiere ein Fell aufweisen.
4. Schaumformulierung zur Verwendung oder Verwendung gemäß einer der Ausführungsformen 1 bis 3, wobei die nicht-menschlichen Säugetiere ausgewählt sind aus der Gruppe bestehend aus Pferden, Eseln, Mauleseln, Maultieren, Rindern, Schweinen, Schafen, Ziegen, Kamelen, Hunden, Katzen, Hasen, Kaninchen und Meerschweinchen, vorzugsweise aus der Gruppe bestehend aus Pferden, Eseln, Mauleseln, Maultieren, Rindern, Schafen, Ziegen, Hunden und Katzen.
5. Schaumformulierung zur Verwendung oder Verwendung gemäß einer der Ausführungsformen 1 bis 4, wobei die Vorbeugung und/oder Behandlung der Hauterkrankung bzw. die kosmetische Pflege das topische Auftragen der Schaumformulierung umfasst.
6. Schaumformulierung zur Verwendung oder Verwendung gemäß einer der Ausführungsformen 1 bis 5, wobei das topische Auftragen der Schaumformulierung einmal, zweimal oder mehrmals täglich, vorzugsweise einmal täglich, erfolgt.
7. Schaumformulierung zur Verwendung oder Verwendung gemäß einer der Ausführungsformen 1 bis 6, wobei die Emulsion Harnstoff umfasst.
8. Schaumformulierung zur Verwendung oder Verwendung gemäß einer der Ausführungsformen 1 bis 7, wobei die Emulsion von 3 bis 30 Gew.-% Harnstoff, bevorzugt von 5 bis 25 Gew.-% Harnstoff, besonders bevorzugt von 5 bis 20 Gew.-% Harnstoff umfasst (jeweils bezogen auf das Gesamtgewicht der Emulsion ohne Treibgas).
9. Schaumformulierung zur Verwendung oder Verwendung gemäß einer der Ausführungsformen 1 bis 8, wobei die Emulsion Mikrosilber, vorzugsweise Mikrosilber und Harnstoff umfasst.
10. Schaumformulierung zur Verwendung oder Verwendung gemäß Ausführungsform 9, wobei die Emulsion von 0,01 bis 3 Gew.-% Mikrosilber, bevorzugt von 0,01 bis 1,5 Gew.-% Mikrosilber, besonders bevorzugt von 0,01 bis 1 Gew.-% Mikrosilber, ganz besonders bevorzugt von 0,01 bis 0,5 Gew.-% Mikrosilber umfasst (jeweils bezogen auf das Gesamtgewicht der Emulsion ohne Treibgas).
11. Schaumformulierung zur Verwendung oder Verwendung gemäß einer der Ausführungsformen 7, 8, 9 oder 10, zur Vorbeugung und/oder Behandlung von, bzw. zur kosmetischen Pflege bei, Mauke, Raspe oder ähnlichen Hauterkrankungen bei Pferden, Eseln, Mauleseln, oder Maultieren, besonders bevorzugt bei Pferden.
12. Schaumformulierung zur Verwendung oder Verwendung gemäß einer der Ausführungsformen 7-11, zur Behandlung von, bzw. zur kosmetischen Pflege bei, Mauke, Raspe oder ähnlichen Hauterkrankungen, wobei die Behandlung bzw. kosmetische Pflege umfasst:
   (a) Behandeln von Krusten auf erkrankten Hautarealen mit einer harnstoffhaltigen wässrigen Lösung;
   (b) Entfernen der in Schritt (a) behandelten Krusten;
   (c) topisches Auftragen der Schaumformulierung auf die erkrankten Hautareale.
13. Schaumformulierung zur Verwendung oder Verwendung gemäß Ausführungsform 12, wobei die harnstoffhaltige wässrige Lösung von 5 bis 35 Gew.-%, bevorzugt von 12 bis 30 Gew.-%, besonders bevorzugt von 15 bis 20 Gew.-% Harnstoff umfasst.
14. Schaumformulierung zur Verwendung oder Verwendung gemäß Ausführungsform 12 oder 13, wobei die harnstoffhaltige wässrige Lösung ferner Allantoin, vorzugsweise in einer Menge von 0,1 bis 5 Gew.-%, umfasst.
15. Schaumformulierung zur Verwendung oder Verwendung gemäß einer der Ausführungsformen 12 bis 14, wobei die harnstoffhaltige wässrige Lösung ferner Panthenol, vorzugsweise in einer Menge von 0,1 bis 5 Gew.-%, umfasst.
16. Schaumformulierung zur Verwendung oder Verwendung gemäß einer der Ausführungsformen 12 bis 15, wobei in Schritt (a) die harnstoffhaltige wässrige Lösung durch Besprühen der erkrankten Hautareale aufgetragen wird.
17. Schaumformulierung zur Verwendung oder Verwendung gemäß einer der Ausführungsformen 1 bis 16, wobei die Emulsion ferner Allantoin umfasst, bevorzugt von 0,01 bis 5 Gew.-% Allantoin, mehr bevorzugt von 0,01 bis 1 Gew.-% Allantoin, besonders bevorzugt von 0,01 bis 0,5 Gew.-% Allantoin umfasst (jeweils bezogen auf das Gesamtgewicht der Emulsion ohne Treibgas).
18. Schaumformulierung zur Verwendung oder Verwendung gemäß einer der Ausführungsformen 1 bis 17, wobei die Emulsion ferner Panthenol umfasst, bevorzugt von 0,2 bis 20 Gew.-% Panthenol, mehr bevorzugt von 0,5 bis 15 Gew.-% Panthenol, besonders bevorzugt von 1 bis 10 Gew.-% Panthenol umfasst (jeweils bezogen auf das Gesamtgewicht der Emulsion ohne Treibgas).
19. Schaumformulierung zur Verwendung oder Verwendung gemäß einer der Ausführungsformen 1 bis 36, wobei die Emulsion ferner Nachtkerzenöl umfasst, bevorzugt von 0,01 bis 8 Gew.-% Nachtkerzenöl, mehr bevorzugt von 0,1 bis 5 Gew.-% Nachtkerzenöl, besonders bevorzugt von 0,5 bis 2,5 Gew.-% Nachtkerzenöl umfasst (jeweils bezogen auf das Gesamtgewicht der Emulsion ohne Treibgas).
20. Schaumformulierung zur Verwendung oder Verwendung gemäß einer der Ausführungsformen 1 bis 19, wobei die Emulsion ferner Aloe Vera-Extrakt umfasst, bevorzugt von 0,01 bis 5 Gew.-% Aloe Vera-Extrakt, mehr bevorzugt von 0,01 bis 3 Gew.-% Aloe Vera-Extrakt, besonders bevorzugt von 0,01 bis 1 Gew.-% Aloe Vera-Extrakt umfasst (jeweils bezogen auf das Trockengewicht des Aloe Vera Extrakts und das Gesamtgewicht der Emulsion ohne Treibgas).
21. Schaumformulierung zur Verwendung oder Verwendung gemäß einer der Ausführungsformen 1 bis 20, wobei die Emulsion ferner Madecassosid umfasst, bevorzugt von 0,01 bis 5 Gew.-% Madecassosid, bevorzugt von 0,01 bis 1 Gew.-% Madecassosid, mehr bevorzugt von 0,01 bis 0,5 Gew.-% Madecassosid umfasst (jeweils bezogen auf das Gesamtgewicht der Emulsion ohne Treibgas).
22. Schaumformulierung zur Verwendung oder Verwendung gemäß einer der Ausführungsformen 1 bis 21, wobei die Emulsion ferner Sorbitol umfasst, bevorzugt von 0,01 bis 8 Gew.-% Sorbitol, mehr bevorzugt von 0,1 bis 5 Gew.-% Sorbitol, besonders bevorzugt von 0,5 bis 2,5 Gew.-% Sorbitol umfasst (jeweils bezogen auf das Gesamtgewicht der Emulsion ohne Treibgas).
23. Schaumformulierung zur Verwendung oder Verwendung gemäß einer der Ausführungsformen 1 bis 22, wobei die Emulsion eine Öl-in-Wasser Emulsion ist.
24. Schaumformulierung zur Verwendung oder Verwendung gemäß einer der Ausführungsformen 1 bis 23, wobei die Schaumformulierung eine Schaumcreme ist.
25. Schaumformulierung zur Verwendung oder Verwendung gemäß einer der Ausführungsformen 1 bis 24, wobei die Schaumformulierung Treibgas, vorzugsweise druckverflüssigtes Treibgas umfasst.
26. Schaumformulierung zur Verwendung oder Verwendung gemäß einer der Ausführungsformen 1 bis 25, wobei das Treibgas ausgewählt ist aus der Gruppe bestehend aus N₂O, Propan, Butan, i-Butan, und Mischungen davon.
27. Schaumformulierung zur Verwendung oder Verwendung gemäß einer der Ausführungsformen 1 bis 26, wobei die Schaumformulierung von 1 bis 20 Gew.-%, bevorzugt von 2 bis 18 Gew.-%, mehr bevorzugt von 5 bis 15 Gew.-% Treibgas enthält.
28. Harnstoff zur Verwendung in der Vorbeugung und/oder Behandlung einer Hauterkrankung bei nicht-menschlichen Säugetieren, wobei die Hauterkrankung ausgewählt ist aus der Gruppe bestehend aus Mauke, Raspe oder ähnlichen Hauterkrankungen.
29. Verwendung von Harnstoff
   a) zur Herstellung eines topischen Medikaments zur Vorbeugung und/oder Behandlung einer Hauterkrankung bei nicht-menschlichen Säugetieren, oder
   b)zur kosmetischen Pflege der Haut nicht-menschlicher Säugetiere bei einer drohenden Hauterkrankung,
   wobei die Hauterkrankung ausgewählt ist aus der Gruppe bestehend Mauke, Raspe oder ähnlichen Hauterkrankungen.
30. Harnstoff zur Verwendung oder Verwendung gemäß Ausführungsform 28 oder 29, wobei der Harnstoff in Form einer Emulsion, einer Lotion, eines Gels, einer Creme, einer Salbe, eines Puders, einer Suspension, einer Lösung, eines Shampoos, einer Tinktur oder einer Schaumformulierung, vorzugsweise in Form einer Schaumformulierung vorliegt.
31. Mikrosilber zur Verwendung in der Vorbeugung und/oder Behandlung einer Hauterkrankung bei nicht-menschlichen Säugetieren, wobei die Hauterkrankung ausgewählt ist aus der Gruppe bestehend aus Mauke, Raspe oder ähnlichen Hauterkrankungen.
32. Verwendung von Mikrosilber
   a) zur Herstellung eines topischen Medikaments zur Vorbeugung und/oder Behandlung einer Hauterkrankung bei nicht-menschlichen Säugetieren, oder
   b) zur kosmetischen Pflege der Haut nicht-menschlicher Säugetiere bei einer drohenden Hauterkrankung,
   wobei die Hauterkrankung ausgewählt ist aus der Gruppe bestehend aus Mauke, Raspe oder ähnlichen Hauterkrankungen.
33. Harnstoff zur Verwendung, Mikrosilber zur Verwendung, oder Verwendung gemäß einer der Ausführungsformen 28 bis 32, zur Vorbeugung und/oder Behandlung von, bzw. zur kosmetischen Pflege bei, Mauke, Raspe oder ähnlichen Hauterkrankungen, bei Pferden, Eseln, Mauleseln, oder Maultieren, bevorzugt bei Pferden.
34. Harnstoff zur Verwendung, Mikrosilber zur Verwendung, oder Verwendung gemäß einer der Ausführungsformen 28-33, zur Behandlung von, bzw. zur kosmetischen Pflege bei, Mauke, Raspe oder ähnlichen Hauterkrankungen, wobei die Behandlung bzw. kosmetische Pflege umfasst:
   (a) Behandeln von Krusten auf erkrankten Hautarealen mit einer harnstoffhaltigen wässrigen Lösung;
   (b) Entfernen der in Schritt (a) behandelten Krusten;
   (c) topisches Auftragen des Harnstoffs bzw. Mikrosilbers auf die erkrankten Hautareale.
35. Mikrosilber zur Verwendung oder Verwendung gemäß einer der Ausführungsformen 31 bis 34, wobei das Mikrosilber in Form einer Emulsion, einer Lotion, eines Gels, einer Creme, einer Salbe, eines Puders, einer Suspension, einer Lösung, eines Shampoos, einer Tinktur oder einer Schaumformulierung, vorzugsweise in Form einer Schaumformulierung vorliegt.
36. Harnstoffhaltige wässrige Lösung, zur Verwendung in der Entfernung von Krusten auf erkrankten Hautarealen von an Mauke, Raspe oder ähnlichen Hauterkrankungen erkrankten nicht-menschlichen Säugetieren, bevorzugt Pferden, Eseln, Mauleseln, oder Maultieren, besonders bevorzugt Pferden.
37. Verwendung einer harnstoffhaltigen wässrigen Lösung
   a) zur Herstellung eines topischen Medikaments, oder
   b) als Kosmetikum,
   zur Entfernung von Krusten auf erkrankten Hautarealen von an Mauke, Raspe oder ähnlichen Hauterkrankungen erkrankten nicht-menschlichen Säugetieren, bevorzugt Pferden, Eseln, Mauleseln, oder Maultieren, besonders bevorzugt Pferden.
38. Harnstoffhaltige wässrige Lösung zur Verwendung oder Verwendung gemäß Ausführungsform 36 oder 37, wobei die harnstoffhaltige wässrige Lösung von 5 bis 35 Gew.-%, bevorzugt von 12 bis 30 Gew.-%, besonders bevorzugt von 15 bis 20 Gew.-% Harnstoff umfasst.
39. Harnstoffhaltige wässrige Lösung zur Verwendung, oder Verwendung gemäß einer der Ausführungsformen 36 bis 38, wobei die harnstoffhaltige wässrige Lösung ferner Allantoin, vorzugsweise in einer Menge von 0,1 bis 5 Gew.-%, umfasst.
40. Harnstoffhaltige wässrige Lösung zur Verwendung, oder Verwendung gemäß einer der Ausführungsformen 36 bis 39, wobei die harnstoffhaltige wässrige Lösung ferner Panthenol, vorzugsweise in einer Menge von 0,1 bis 5 Gew.-%, umfasst.
41. Harnstoffhaltige wässrige Lösung zur Verwendung, oder Verwendung gemäß einer der Ausführungsformen 36 bis 40, wobei die harnstoffhaltige wässrige Lösung in Form einer Sprayformulierung vorliegt, die vorzugsweise zusätzlich ein (druck)verflüssigtes Treibgas enthält.
42. Kit umfassend
   a) eine harnstoffhaltige wässrige Lösung, und
   b) eine Schaumformulierung umfassend eine Emulsion, umfassend eine Ölphase und eine Wasserphase, wobei die Emulsion Mikrosilber und/oder Harnstoff umfasst.
43. Kit gemäß Ausführungsform 42, zur Verwendung in der Behandlung von Mauke, Raspe oder ähnlichen Hauterkrankungen, bevorzugt bei Pferden, Eseln, Mauleseln, oder Maultieren, besonders bevorzugt bei Pferden.

### 6. Beispiele

### 6.1. Beispiel 1: Behandlung von Mauke beim Pferd

In einer klinischen Studie wurde eine Mikrosilber-haltige Schaumcreme folgender Zusammensetzung zur Behandlung von Mauke beim Pferd untersucht:

| **Zusammensetzung "Schaumcreme Mikrosilber":** | | |
|---|---|---|
| **Inhaltsstoff** | **INCI-Name** | **Gew.-%** |
| Cetiol V | Decyloleat | 5,00 |
| Eutanol G | Octyldodecanol | 5,00 |
| Cutina MD | Glycerylstearat | 2,00 |
| Lanette O | Cetylstearylalkohol | 4,00 |
| Edenor C 18 98/100 | Stearinsäure | 2,50 |
| Nanocream (Emulgator) | Kaliumlauroyl-Weizenaminosäuren, Palmglyceride, Capryloylglycin | 2,00 |
| demin. Wasser | | 64,53 |
| Propylenglykol 1,2 | | 2,50 |
| Glycerin 86% | | 2,50 |
| Protelan LS 9011 | Natrium-Lauroylsarkosinat | 2,00 |
| Allantoin Pulver | | 0,10 |
| Madecassoside | | 0,10 |
| D-Panthenol 75L | | 6,67 |
| Nachtkerzenöl | | 1,00 |
| Mikrosilber | | 0,10 |
| **Gesamt** | | **100,00** |

Insgesamt 25 Pferde mit Mauke wurden mit der "Schaumcreme Mikrosilber" behandelt. Entsprechend der Hautveränderungen erfolgte eine Unterteilung in drei Gruppen:

| | |
|---|---|
| **Gruppe I:** | Geringgradige Rötung und oberflächliche Reizung bzw. Irritation der Haut |
| **Gruppe II:** | Entzündliche Hautveränderung mit geringgradiger Exsudation |
| **Gruppe III:** | Trockene Mauke mit Krusten und Borken |

### a) Ergebnisse Gruppe I

In die Gruppe I wurden alle Patienten aufgenommen, die leichte Hautirritationen in Form von Rötung der Haut oder leichter Rissbildung zeigten. Zu dieser Gruppe zählten acht Pferde.

Die Behandlung erfolgte durch ein- bis zweimal tägliches Auftragen der "Schaumcreme Mikrosilber" auf die betroffenen Hautareale. Bei allen acht behandelten Pferden kamen die genannten Symptome innerhalb von 1- 2 Wochen zur vollständigen Abheilung.

### b) Ergebnisse Gruppe II

In die Gruppe II wurden alle Pferde aufgenommen, die eine entzündliche, nässende Mauke zeigten. Zu dieser Gruppe zählten sechs Pferde.

Die Behandlung erfolgte durch ein- bis zweimal tägliches Auftragen der "Schaumcreme Mikrosilber" auf die betroffenen Hautareale.
Zwei der Patienten wurden vorab mit mildem desinfizierenden Mittel (Jodseife) gewaschen und mit antibiotischen Salben vorbehandelt.

Bei fünf Pferden konnte eine vollständige Abheilung der Veränderungen erreicht werden. Zu dieser Gruppe gehörten die mit Iodseife/antibiotischer Salbe vorbehandelten Patienten. Lediglich bei einem Pferd blieb die Schaumcreme-Behandlung ohne Erfolg.

### c) Ergebnisse Gruppe III

In die Gruppe III wurden elf Pferde eingeordnet, die trockene, krustige oder borkige Formen von Mauke zeigten.

Je nach Schweregrad wurde initial eine Behandlung mit keratolytisch wirksamen Präparaten vorgenommen, um Krusten und Borken zu lösen. Im Anschluss daran wurde ein- bis zweimal täglich die "Schaumcreme Mikrosilber" auf die betroffenen Hautareale aufgetragen.

Bei vier Pferden kamen die Veränderungen in einem Beobachtungszeitraum von 14 Tagen zur Abheilung, bei zwei weiteren Patienten verbesserte sich das klinische Bild deutlich. Bei fünf Pferden zeigte die Behandlung mit "Schaumcreme Mikrosilber" keinen Effekt. Hierbei handelte es sich ausnahmslos um Pferde, bei denen eine keratolytische Vorbehandlung unterblieben war und der Schaum auf die Borken aufgetragen wurde, so dass die Wirkstoffe die Haut nicht oder nur teilweise erreichen konnten.

### Keratolytische Vorbehandlung:

Die oben beschriebene keratolytische Vorbehandlung erfolgte bei drei Patienten durch je nach Bedarf ein- oder mehrmaliges Besprühen der Borken und Krusten mit einer Sprühvorrichtung enthaltend eine harnstoffhaltige wässrige Lösung folgender Zusammensetzung:

| **Basiszusammensetzung Keratolytikum:** | |
|---|---|
| **Inhaltsstoff** | **Gew.-%** |
| demin. Wasser | 80,10 |
| Harnstoff | 18,00 |
| Milchsäure 90% | 0,22 |
| Euxyl PE 9010 (Konservierungsmittel, basierend auf Phenoxyethanol und Ethylhexylglycerin) | 1,00 |
| D-Panthenol 75 L | 0,40 |
| Allantoin | 0,20 |
| NaOH-Plätzchen (100%) | 0,08 |
| **Gesamt** | **100,00** |

Hierbei konnte bei den behandelten Pferden beobachtet werden, dass die harten, die veränderten Hautareale bedeckenden Borken weich wurden. In zwei Fällen wurde die Anwendung nach zwei Tagen wiederholt, da eine einmalige Anwendung nicht ausreichend war.

### 6.2. Beispiel 2: Wundbehandlung beim Pferd

In einer klinischen Studie wurde die Mikrosilber-haltige Schaumcreme aus Beispiel 1 zur Wundbehandlung beim Pferd untersucht.

Eingesetzt wurde die "Schaumcreme Mikrosilber" bei acht Pferden in der Behandlung von oberflächlichen Schürfwunden sowie in der Nachbehandlung von größeren Wunden bzw. Dekubitusstellen. Zudem wurde die Schaumcreme zur Behandlung gereizter Fohlenhaut im Bereich der Hinterschenkel infolge starker Fohlendiarrhoe verwendet. Bei offenen Wunden kam die Schaumcreme nicht zum Einsatz.

Die Behandlung erfolgte durch ein- bis zweimal tägliches Auftragen der "Schaumcreme Mikrosilber" auf die betroffenen Hautareale.

Bei allen Patienten konnte bereits nach wenigen Tagen eine deutliche Verbesserung der Hautelastizität erreicht und ein rasches Nachwachsen der Haare beobachtet werden.

### 6.3. Beispiel 3: Behandlung von Ekzemen beim Pferd

In einer klinischen Studie wurde eine Harnstoff-haltige Schaumcreme folgender Zusammensetzung zur Behandlung von Ekzemen beim Pferd untersucht.

| **Zusammensetzung "Schaumcreme Harnstoff":** | | |
|---|---|---|
| **Inhaltsstoff** | **INCI-Name** | **Gew.-%** |
| Cetiol V | Decyloleat | 7,00 |
| Eutanol G | Octyldodecanol | 7,00 |
| Cutina MD | Glycerylstearat | 2,00 |
| Lanette O | Cetylstearylalkohol | 4,00 |
| Edenor C 18 98/100 | Stearinsäure | 2,50 |
| Nanocream (Emulgator) | Kaliumlauroyl-Weizen Aminosäuren, Palmglyceride, Capryloylglycin | 3,00 |
| demin. Wasser | | 48,80 |
| Harnstoff | | 16,50 |
| Propylenglykol 1,2 | | 2,50 |
| Glycerin 86% | | 2,50 |
| Protelan LS 9011 | Natrium-Lauroylsarkosinat | 2,00 |
| Allantoin Pulver | | 0,10 |
| Sorbitol (Karion FP) | | 1,00 |
| Aloe Vera sprühgetrocknet 200:1 | | 0,10 |
| Nachtkerzenöl gepresst DAC | | 1,00 |
| **Gesamt** | | **100,00** |

Insgesamt 24 Pferde wurden mit der "Schaumcreme Harnstoff" behandelt. Allen Pferden war gemein, dass sie unter Juckreiz litten. Milben oder andere Parasiten konnten in keinem Fall nachgewiesen werden.

Die Patienten wurden in zwei Gruppen eingeteilt.

| | |
|---|---|
| **Gruppe I:** | Zur Gruppe I zählten 22 Pferde, die an einem trockenen, schuppigen Ekzem litten, das überwiegend im Bereich des Mähnenansatzes und der Schweifrübe lokalisiert war. |
| **Gruppe II:** | Zur Gruppe II zählten zwei Pferde, bei denen die Hautveränderungen nässend und/ oder blutig waren. |

### a) Ergebnisse Gruppe I

Bei den Patienten der Gruppe I wurde ein- bis zweimal täglich die "Schaumcreme Harnstoff" auf die betroffenen Hautareale aufgetragen.

Bei elf Pferden wurde beobachtet, dass der Juckreiz bereits wenige Tage nach der ersten Behandlung mit der "Schaumcreme Harnstoff" komplett abgeklungen war und die Haut eine deutlich verbesserte Geschmeidigkeit zeigte. Die Veränderungen waren nach 1-2 Wochen vollständig abgeheilt. Bei weiteren sechs Pferden verbesserte sich das Krankheitsbild erheblich. Fünf Pferde aus Gruppe I erwiesen sich als therapieresistent.

### b) Ergebnisse Gruppe II

Bei den Patienten der Gruppe II wurden die veränderten Hautareale initial mit Jodseife gewaschen. Anschließend wurde ein- bis zweimal täglich die "Schaumcreme Harnstoff" auf die betroffenen Hautpartien aufgetragen.

Auch hier wurde eine vollständige Abheilung der Hautveränderungen beobachtet.

Für beide Gruppen galt, dass die Schaumcreme täglich auch über das Abklingen der Symptome hinaus aufgetragen werden musste, um den Juckreiz dauerhaft zu unterbinden und die Haut geschmeidig zu halten.

### 6.4. Zusammenfassende Beurteilung

Die untersuchten Schaumcremes führten zu einer deutlichen Verbesserung der Hautelastizität und -geschmeidigkeit, wodurch die Abheilung von Wunden gefördert, als auch der Rezidivbildung von Dermatitiden oder Ekzemen vorgebeugt wird.

Die "Schaumcreme Harnstoff" zeichnete sich dabei durch juckreizlindernden Eigenschaft aus, wodurch der Kreislauf zwischen scheuerbedingten Hautirritationen, die wiederum zu vermehrtem Juckreiz führen, wirkungsvoll unterbrochen werden konnte.

## Patentansprüche

1. Schaumformulierung umfassend eine Emulsion, umfassend eine Ölphase und eine Wasserphase, wobei die Emulsion Mikrosilber und/oder Harnstoff umfasst, zur Verwendung in der Vorbeugung und/oder Behandlung einer Hauterkrankung bei nicht-menschlichen Säugetieren, wobei die Hauterkrankung ausgewählt ist aus der Gruppe bestehend aus Mauke, Raspe oder ähnlichen Hauterkrankungen.

2. Verwendung einer Schaumformulierung umfassend eine Emulsion, umfassend eine Ölphase und eine Wasserphase, wobei die Emulsion Mikrosilber und/oder Harnstoff umfasst, zur kosmetischen Pflege der Haut nicht-menschlicher Säugetiere bei einer drohenden Hauterkrankung, wobei die Hauterkrankung ausgewählt ist aus der Gruppe bestehend aus Mauke, Raspe oder ähnlichen Hauterkrankungen.

3. Schaumformulierung oder Verwendung gemäß Anspruch 1 oder 2, wobei die Emulsion Mikrosilber und Harnstoff umfasst.

4. Schaumformulierung zur Verwendung oder Verwendung gemäß einem der Ansprüche 1 bis 3, wobei die Emulsion von 3 bis 30 Gew.-% Harnstoff, bevorzugt von 5 bis 25 Gew.-% Harnstoff, besonders bevorzugt von 5 bis 20 Gew.-% Harnstoff umfasst (jeweils bezogen auf das Gesamtgewicht der Emulsion ohne Treibgas).

5. Schaumformulierung zur Verwendung oder Verwendung gemäß einem der Ansprüche 1 bis 4, wobei die Emulsion von 0,01 bis 3 Gew.-% Mikrosilber, bevorzugt von 0,01 bis 1,5 Gew.-% Mikrosilber, besonders bevorzugt von 0,01 bis 1 Gew.-% Mikrosilber, ganz besonders bevorzugt von 0,01 bis 0,5 Gew.-% Mikrosilber umfasst (jeweils bezogen auf das Gesamtgewicht der Emulsion ohne Treibgas).

6. Schaumformulierung zur verwendung oder Verwendung gemäß einem der Ansprüche 1 bis 5, zur Verwendung in der Vorbeugung und/oder Behandlung von, bzw. zur kosmetischen Pflege bei, Mauke, Raspe oder ähnlichen Hauterkrankungen bei Pferden, Eseln, Mauleseln, oder Maultieren, bevorzugt bei Pferden.

7. Schaumformulierung zur Verwendung oder Verwendung gemäß einem der vorangehenden Ansprüche, zur Verwendung in der Behandlung von, bzw. zur kosmetischen Pflege bei, Mauke, Raspe oder ähnlichen Hauterkrankungen, wobei die Behandlung bzw. kosmetische Pflege umfasst:
(a) Behandeln von Krusten auf erkrankten Hautarealen mit einer harnstoffhaltigen wässrigen Lösung;
(b) Entfernen der in Schritt (a) behandelten Krusten;
(c) topisches Auftragen der Schaumformulierung auf die erkrankten Hautareale.

8. Schaumformulierung zur Verwendung oder Verwendung gemäß Anspruch 7, wobei die harnstoffhaltige wässrige Lösung von 5 bis 35 Gew.-%, bevorzugt von 12 bis 30 Gew.-%, besonders bevorzugt von 15 bis 20 Gew.-% Harnstoff umfasst.

9. Schaumformulierung zur Verwendung oder Verwendung gemäß Anspruch 7 oder 8, wobei in Schritt (a) die harnstoffhaltige wässrige Lösung durch Besprühen der erkrankten Hautareale aufgetragen wird.

10. Harnstoff zur Verwendung in der Vorbeugung und/oder Behandlung einer Hauterkrankung bei nicht-menschlichen Säugetieren, wobei die Hauterkrankung ausgewählt ist aus der Gruppe bestehend aus Mauke, Raspe oder ähnlichen Hauterkrankungen.

11. Mikrosilber zur Verwendung in der Vorbeugung und/oder Behandlung einer Hauterkrankung bei nicht-menschlichen Säugetieren, wobei die Hauterkrankung ausgewählt ist aus der Gruppe bestehend aus Mauke, Raspe, oder ähnlichen Hauterkrankungen.

12. Harnstoffhaltige wässrige Lösung, zur Verwendung in der Entfernung von Krusten auf erkrankten Hautarealen von an Mauke, Raspe oder ähnlichen Hauterkrankungen erkrankten nicht-menschlichen Säugetieren.

13. Verwendung einer harnstoffhaltigen wässrigen Lösung als Kosmetikum zur Entfernung von Krusten auf erkrankten Hautarealen von an Mauke, Raspe oder ähnlichen Hauterkrankungen erkrankten nicht-menschlichen Säugetieren.

14. Harnstoffhaltige wässrige Lösung oder Verwendung gemäß Anspruch 12 oder 13, wobei die harnstoffhaltige wässrige Lösung von 5 bis 35 Gew.-%, bevorzugt von 12 bis 30 Gew.-%, besonders bevorzugt von 15 bis 20 Gew.-% Harnstoff umfasst.

15. Kit umfassend
a) eine harnstoffhaltige wässrige Lösung, und
b) eine Schaumformulierung umfassend eine Emulsion, umfassend eine Ölphase und eine Wasserphase, wobei die Emulsion Mikrosilber und/oder Harnstoff umfasst.

## Claims

1. Foam formulation comprising an emulsion, comprising an oil phase and an aqueous phase, wherein the emulsion comprises microsilver and/or urea, for use in the prevention and/or the treatment of a skin disease in non-human mammals, wherein the skin disease is selected from the group consisting of fetlock eczema (greasy heel, "Mauke"), cracked hock ("Raspe"), or similar skin diseases.

2. Use of a foam formulation comprising an emulsion, comprising an oil phase and an aqueous phase, wherein the emulsion comprises microsilver and/or urea, for the cosmetic care of the skin of non-human mammals in the case of an imminent skin disease, wherein the skin disease is selected from the group consisting of fetlock eczema (greasy heel), cracked hock, or similar skin diseases.

3. Foam formulation or use according to claim 1 or 2, wherein the emulsion comprises microsilver and urea.

4. Foam formulation for use or use according to any one of claims 1 to 3, wherein the emulsion comprises from 3 to 30 % (by wt) of urea, preferably from 5 to 25 % (by wt) of urea, particularly preferred from 5 to 20 % (by wt) of urea (each based on the total weight of the emulsion without propellant).

5. Foam formulation for use or use according to any one of claims 1 to 4, wherein the emulsion comprises from 0.01 to 3 % (by wt) of microsilver, preferably from 0.01 to 1.5 % (by wt) of microsilver, particularly preferred from 0.01 to 1 % (by wt) of microsilver, more particularly preferred from 0.01 to 0.5 % (by wt) of microsilver (each based on the total weight of the emulsion without propellant).

6. Foam formulation for use or use according to any one of claims 1 to 5, for use in the prevention and/or the treatment of, or for the cosmetic care in the case of, fetlock eczema (greasy heel), cracked hock, or similar skin diseases, in horses, donkeys, hinnies, or mules, preferably in horses.

7. Foam formulation for use or use according to any one of the preceding claims, for use in the treatment of, or for the cosmetic care in the case of, fetlock eczema (greasy heel), cracked hock or similar skin diseases, wherein the treatment or the cosmetic care comprises:
(a) treatment of crusts on diseased skin areas with an aqueous solution containing urea;
(b) removal of the crusts treated in step (a);
(c) topical application of the foam formulation onto the diseased skin areas.

8. Foam formulation for use or use according to claim 7, wherein the aqueous solution containing urea comprises from 5 to 35 % (by wt), preferably from 12 to 30 % (by wt), particularly preferred from 15 to 20 % (by wt) of urea.

9. Foam formulation for use or use according to claim 7 or 8, wherein in step (a) the aqueous solution containing urea is applied by spraying the diseased skin areas.

10. Urea for use in the prevention and/or the treatment of a skin disease in non-human mammals, wherein the skin disease is selected from the group consisting of fetlock eczema (greasy heel), cracked hock or similar skin diseases.

11. Microsilver for use in the prevention and/or the treatment of a skin disease in non-human mammals, wherein the skin disease is selected from the group consisting of fetlock eczema (greasy heel), cracked hock, or similar skin diseases.

12. Aqueous solution containing urea, for use in the removal of crusts on diseased skin areas of non-human mammals suffering from fetlock eczema (greasy heel), cracked hock, or similar skin diseases.

13. Use of an aqueous solution containing urea as cosmetic for the removal of crusts on diseased skin areas of non-human mammals suffering from fetlock eczema (greasy heel), cracked hock, or similar skin diseases.

14. Aqueous solution containing urea or use according to claim 12 or 13, wherein the aqueous solution containing urea comprises from 5 to 35 % (by wt), preferably from 12 to 30 % (by wt), particularly preferred from 15 to 20 % (by wt) of urea.

15. Kit comprising
a) an aqueous solution containing urea, and
b) a foam formulation comprising an emulsion, comprising an oil phase and an aqueous phase, wherein the emulsion comprises microsilver and/or urea.

## Revendications

1. Formulation de mousse comprenant une émulsion, comprenant une phase huileuse et une phase aqueuse, dans laquelle l'émulsion comprend des microparticules d'argent et/ou de l'urée, destinée à être utilisée dans la prévention et/ou le traitement d'une maladie cutanée chez les mammifères non humains, dans laquelle la maladie cutanée est sélectionnée dans le groupe constitué par l'eczéma du paturon (malandre, « Mauke »), les crevasses au pli du jarret (« Raspe »), ou des maladies cutanées similaires.

2. Utilisation d'une formulation de mousse comprenant une émulsion, comprenant une phase huileuse et une phase aqueuse, dans laquelle l'émulsion comprend des microparticules d'argent et/ou de l'urée, destinée aux soins cosmétiques de la peau de mammifères non humains dans le cas d'une maladie cutanée imminente, dans laquelle la maladie cutanée est sélectionnée dans le groupe constitué par l'eczéma du paturon (malandre), les crevasses au pli du jarret, ou des maladies cutanées similaires.

3. Formulation de mousse ou utilisation selon la revendication 1 ou 2, dans laquelle l'émulsion comprend des microparticules d'argent et de l'urée.

4. Formulation de mousse destinée à une utilisation ou utilisation selon l'une quelconque des revendications 1 à 3, dans laquelle l'émulsion comprend de 3 à 30 % (en poids) d'urée, de préférence de 5 à 25 % (en poids) d'urée, de manière particulièrement préférée de 5 à 20 % (en poids) d'urée (à chaque fois sur la base du poids total de l'émulsion sans agent propulseur).

5. Formulation de mousse destinée à une utilisation ou utilisation selon l'une quelconque des revendications 1 à 4, dans laquelle l'émulsion comprend de 0,01 à 3 % (en poids) de microparticules d'argent, de préférence de 0,01 à 1,5 % (en poids) de microparticules d'argent, de manière particulièrement préférée de 0,01 à 1 % (en poids) de microparticules d'argent, de manière plus particulièrement préférée de 0,01 à 0,5 % (en poids) de microparticules d'argent (à chaque fois sur la base du poids total de l'émulsion sans agent propulseur).

6. Formulation de mousse destinée à une utilisation ou utilisation selon l'une quelconque des revendications 1 à 5, destinée à être utilisée dans la prévention et/ou le traitement, ou destinée aux soins cosmétiques dans le cas, de l'eczéma du paturon (malandre), des crevasses au pli du jarret, ou de maladies cutanées similaires, chez les chevaux, les singes, les bardots, ou les mules, de préférence chez les chevaux.

7. Formulation de mousse destinée à une utilisation ou utilisation selon l'une quelconque des revendications précédentes, destinée à être utilisée dans le traitement, ou destinée aux soins cosmétiques dans le cas, de l'eczéma du paturon (malandre), des crevasses au pli du jarret, ou de maladies cutanées similaires, dans laquelle le traitement ou les soins cosmétiques comprennent :
(a) le traitement de croûtes sur des zones cutanées atteintes avec une solution aqueuse contenant de l'urée ;
(b) l'élimination des croûtes traitées à l'étape (a) ;
(c) l'application topique de la formulation de mousse sur les zones cutanées atteintes.

8. Formulation de mousse destinée à une utilisation ou utilisation selon la revendication 7, dans laquelle la solution aqueuse contenant de l'urée comprend de 5 à 35 % (en poids), de préférence de 12 à 30 % (en poids), de manière particulièrement préférée de 15 à 20 % (en poids) d'urée.

9. Formulation de mousse destinée à une utilisation ou utilisation selon la revendication 7 ou 8, dans laquelle à l'étape (a) la solution aqueuse contenant de l'urée est appliquée par pulvérisation sur les zones cutanées atteintes.

10. Urée destinée à être utilisée dans la prévention et/ou le traitement d'une maladie cutanée chez des mammifères non humains, la maladie cutanée étant sélectionnée dans le groupe constitué par l'eczéma du paturon (malandre), les crevasses au pli du jarret ou des maladies cutanées similaires.

11. Microparticules d'argent destinées à être utilisées dans la prévention et/ou le traitement d'une maladie cutanée chez des mammifères non humains, la maladie cutanée étant sélectionnée dans le groupe constitué par l'eczéma du paturon (malandre), les crevasses au pli du jarret ou des maladies cutanées similaires.

12. Solution aqueuse contenant de l'urée, destinée à être utilisée dans l'élimination de croûtes sur des zones cutanées atteintes de mammifères non humains souffrant d'eczéma du paturon (malandre), de crevasses au pli du jarret ou de maladies cutanées similaires.

13. Utilisation d'une solution aqueuse contenant de l'urée en tant que produit cosmétique pour l'élimination de croûtes sur des zones cutanées atteintes de mammifères non humains souffrant d'eczéma du paturon (malandre), de crevasses au pli du jarret ou de maladies cutanées similaires.

14. Solution aqueuse contenant de l'urée ou utilisation selon la revendication 12 ou 13, dans laquelle la solution aqueuse contenant de l'urée comprend 5 à 35 % (en poids), de préférence de 12 à 30 % (en poids), de manière particulièrement préférée de 15 à 20 % (en poids) d'urée.

15. Kit comprenant
a) une solution aqueuse contenant de l'urée, et
b) une formulation de mousse comprenant une émulsion, comprenant une phase huileuse et une phase aqueuse, dans laquelle l'émulsion comprend des microparticules d'argent et/ou de l'urée.
